(19) Europäisches Patentamt
European Patent Office
Office européen des brevets

(11) Veröffentlichungsnummer: **0 031 875**
**B1**

(12) **EUROPÄISCHE PATENTSCHRIFT**

(45) Veröffentlichungstag der Patentschrift: **18.05.83**

(51) Int. Cl.³: **C 07 C 175/00**

(21) Anmeldenummer: **80105995.7**

(22) Anmeldetag: **03.10.80**

(54) **Verfahren zur Herstellung von Cyclohexenderivaten.**

(30) Priorität: **28.11.79 CH 10581/79**

(43) Veröffentlichungstag der Anmeldung: **15.07.81 Patentblatt 81/28**

(45) Bekanntmachung des Hinweises auf die Patenterteilung: **18.05.83 Patentblatt 83/20**

(84) Benannte Vertragsstaaten: **AT BE CH DE FR GB IT LI NL**

(56) Entgegenhaltungen:
**EP-A-0 005 748**
**US-A-4 153 805**

(73) Patentinhaber: **F. HOFFMANN-LA ROCHE & CO. Aktiengesellschaft, CH-4002 Basel (CH)**

(72) Erfinder: **Lukàc, Teodor, Steinackerstrasse 44, CH-4147 Aesch (CH)**
Erfinder: **Widmer, Erich, Dr., Mittelweg 47, CH-4142 Münchenstein (CH)**
Erfinder: **Zell, Reinhard, Dr., Im Zwären, CH-4148 Rodersdorf (CH)**

(74) Vertreter: **Cottong, Norbert A. et al, *Grenzacherstrasse 124 Postfach 3255, CH-4002 Basel (CH)***

EP 0 031 875 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Cyclohexenderivaten

Die vorliegende Erfindung betrifft ein Verfahren zur Herstellung von Cyclohexenderivaten, welche als Zwischenprodukte für die Herstellung von Canthaxanthin geeignet sind, sowie ein Verfahren zur Herstellung von Canthaxanthin selbst.

Das erfindungsgemässe Verfahren ist dadurch gekennzeichnet, dass man eine Verbindung der Formel

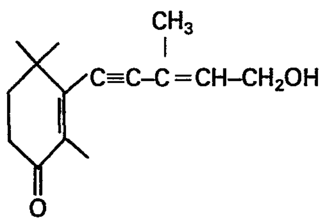

I

mittels Zink und Ameisensäure, Essigsäure oder Propionsäure hydriert und gewünschtenfalls die dabei erhaltene Verbindung der Formel

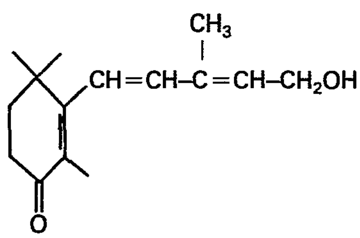

II

nach Überführung in ein Phosphoniumsalz der Formel

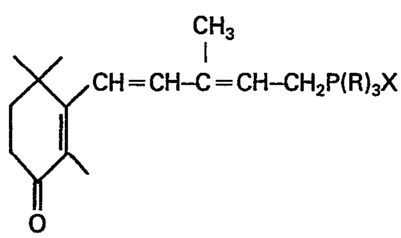

III

worin R Phenyl und X Chlor, Brom oder Jod bedeutet, in Canthaxanthin überführt.

In US-A-4 153 805 wird die Umsetzung der Verbindung der Formel I zur Verbindung der Formel II durch katalytische Hydrierung und die weitere Überführung in Canthaxanthin beschrieben.

Der erste Schritt des erfindungsgemässen Verfahrens, nämlich die partielle Hydrierung der Verbindung der Formel I zur Verbindung der Formel II, erfolgt, wie oben ausgeführt, mittels Zink und einer Carbonsäure mit bis zu 3 Kohlenstoffatomen, nämlich Ameisensäure, Essigsäure oder Propionsäure.

Eine besonders zweckmässige Ausführungsform besteht darin, dass man die Hydrierung mit Zink und Eisessig durchführt.

Hierbei wird zweckmässig in einem geeigneten organischen Lösungsmittel, beispielsweise einem chlorierten Kohlenwasserstoff, wie z.B. Methylenchlorid, gearbeitet. Es ist jedoch auch möglich, die Hydrierung in Abwesenheit eines derartigen Lösungsmittels durchzuführen, in welchem Falle dann die Säure selbst als Lösungsmittel dient.

Vorzugsweise verwendet man eine etwa 2%ige Lösung des Ausgangsmaterials der Formel I, bezogen auf das Gemisch von Lösungsmittel und Säure. Das Verhältnis von Lösungsmittel zur Säure, also beispielsweise das Verhältnis von Methylenchlorid zu Eisessig beträgt zweckmässig etwa 1:2 bis 1:2,5.

Zwecks vollständiger Durchführung der Partialhydrierung sollten Zink und Säure in mindestens stöchiometrischen Mengen in Bezug auf das Ausgangsmaterial der Formel I verwendet werden, jedoch ist die Verwendung eines Zink- und Säureüberschusses bevorzugt.

Das Zink wird zweckmässig in einer Menge von etwa 1–3 Grammatom, vorzugsweise 1,5 Grammatom pro Mol Ausgangsmaterial verwendet.

Die für die Hydrierung gewählte Reaktionstemperatur kann innerhalb eines weiten Temperaturbereiches liegen, beispielsweise zwischen etwa −20 °C und etwa Raumtemperatur, wobei die Durchführung der Reaktion bei etwa 0 °C bevorzugt ist.

Bei der Überführung der Verbindung der Formel II in ein Phosphoniumsalz der Formel III, wird die Verbindung der Formel II vorerst in an sich bekannter Weise mittels eines Halogenwasserstoffs (Chlorwasserstoff, Bromwasserstoff oder Jodwasserstoff) in wässriger Lösung (z.B. 48%. 63% oder 57%) halogeniert. Diese Halogenierung kann bei Temperaturen zwischen etwa −10 °C und etwa +10 °C, vorzugsweise bei etwa 0 °C, durchgeführt werden. Als Lösungsmittel verwendet man hierbei ein für derartige Halogenierungen geeignetes Lösungsmittel, biespielsweise einen chlorierten Kohlenwasserstoff, wie z.B. Methylenchlorid oder Chloroform.

Das so erhaltene Halogenid wird hierauf mit einem Triarylphosphin, insbesondere Triphenylphosphin, in an sich bekannter Weise in ein Phosphoniumsalz der Formel III übergeführt, beispielsweise in Äthylacetat, vorzugsweise in einer inerten Gasatmosphäre und in Gegenwart eines Säure bindenden Mittels, z.B. eines Alkylenoxids, wie 1,2-Butylenoxid, zweckmässig bei Raumtemperatur oder etwas erhöhter Temperatur.

Bei der Herstellung von Canthaxanthin aus dem Phosphoniumsalz der Formel III wird letzteres mit dem Dialdehyd 2,7-Dimethyl-octatrien-(2,4,6)-dial-(1,8) umgesetzt, wobei man, ebenfalls zweckmässig in einem geeigneten Lösungsmittel, beispielsweise in Methylenchlorid oder Chloroform, und in Anwesenheit eines säurebindenden Mittels, beispielsweise von 1,2-Butylenoxid oder Natriummethylat, arbeitet.

Die in dem erfindungsgemässen Verfahren als Ausgangsmaterial verwendete Verbindung der Formel I ist bekannt und kann nach bekannten Methoden hergestellt werden. Sie kann jedoch auch dadurch erhalten werden, dass man eine Verbindung der Formel

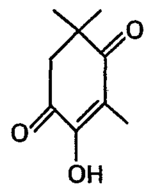

IV

mittels Zink und einer Carbonsäure mit bis zu 3 Kohlenstoffatomen, nämlich mit Ameisensäure, Essigsäure oder Propionsäure, zu einer Verbindung der Formel

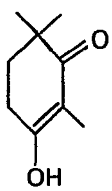

V

reduziert, die freie Hydroxygruppe dieser Verbindung in eine geschützte Hydroxygruppe überführt und die so erhaltene Verbindung unter Bildung einer Verbindung der Formel I alkinyliert.

Beim ersten Schritt dieses Verfahrens, der Reduktion der Verbindung der Formel IV, wird zweckmässig ebenfalls in einem geeigneten Lösungsmittel, insbesondere einem chlorierten Kohlenwasserstoff, z.B. Äthylenchlorid, gearbeitet. Auch hier kann jedoch in Abwesenheit eines solchen Lösungsmittels gearbeitet werden, wobei dann die Säure selbst als Lösungsmittel fungiert. Zweckmässig wird das Ausgangsmaterial in einer Konzentration von etwa 5–15%, beispielsweise etwa 10%, verwendet. Das Zink kann beispielsweise in einer Menge von etwa 3 Grammatom pro Mol Ausgangsmaterial verwendet werden. Zweckmässige Reaktionstemperaturen liegen zwischen etwa 45 °C und 80 °C, wobei eine Temperatur von etwa 75 °C bevorzugt ist.

Eine besonders bevorzugte Ausführungsform besteht darin, dass man die Reduktion mit Zink und Ameisensäure durchführt.

Das erhaltene Produkt stellt ein Tautomerengemisch von 3-Hydroxy-2,6,6-trimethyl-2-cyclohexen-1-on (Verbindung V) und 3-Hydroxy-2,4,4-trimethyl-2-cyclohexen-1-on dar.

Hierauf wird die freie Hydroxygruppe der Verbindung der Formel V, beispielsweise durch Bildung eines Niederalkyläthers, z.B. des Äthyläthers, in an sich bekannter Weise geschützt und der so erhaltene Äther mit einem geschützten 3-Methyl-2-penten-4-in, beispielsweise dem Aceton-methyl-(3-methyl-2-penten-4-inyl)-acetat alkinyliert. Diese Alkinylierung wird zweckmässig in einem Lösungsmittel, beispielsweise Tetrahydrofuran, unter Verwendung von Butyllithium in an sich bekannter Weise durchgeführt, wobei das primär gebildete Addukt sauer aufgearbeitet und zur Verbindung der Formel I hydrolysiert wird.

Die hierin enthaltenen Formeln sind ohne Berücksichtigung der cis/trans-Isomerie dargestellt. Vorzugsweise handelt es sich um all-trans-Verbindungen. Bei der Partialhydrierung der Formel I entsteht die Verbindung der Formel II überwiegend in der 7-cis-Form. Bei der Überführung der Verbindung der Formel II in das Phosphoniumsalz III entsteht dieses unter den beschriebenen Reaktionsbedingungen wie üblich in all-trans-Form.

Beispiel 1

In einem 2,5-Liter-Sulfierkolben, versehen mit Rührer, Thermometer, gefülltem Chlorcalciumrohr und einer Vorrichtung zur Inertbegasung werden unter Rühren und Begasung mit Argon 46,3 g 5-(2,6,6-Trimethyl-3-oxo-cyclohexen-1-yl)-3-methyl-2-trans-penten-4-in-1-ol in 1400 ml Methylenchlorid und 300 ml Essigsäure gelöst. Darauf wird mit einem Alkohol/Trockeneis-Kühlbad auf 0 °C gekühlt und 23,0 g Zinkstaub zugegeben und unter Argon während 3 Stunden bei 0 °C weiter gerührt.

Das noch kalte Reaktionsgemisch wird über eine Glassinternutsche filtriert und der Rückstand auf der Nutsche mit 2 Portionen zu je 100 ml, insgesamt also mit 200 ml, Methylenchlorid gewaschen.

Von vier 3-Liter-Scheidetrichtern ($S_1$–$S_4$) werden $S_1$ mit 700 ml Eiswasser, $S_2$ und $S_3$ mit je 1000 ml, insgesamt also mit 2000 ml gesättigter Natriumbicarbonatlösung und $S_4$ mit 500 ml Wasser (entionisiert) beschickt. Nun werden das filtrierte Reaktionsgemisch und anschliessend zwei Portionen zu je 300 ml, insgesamt also 600 ml, Methylenchlorid der Reihe nach und unter jeweiligem kräftigem Schütteln durch die vier Scheidetrichter $S_1$–$S_4$ geschickt. Die organischen Phasen werden vereinigt, über 150 g Natriumsulfat getrocknet, filtriert und im Rotationsverdampfer am Wasserstrahlvakuum bei einer Badtemperatur von 50 °C bis zur Gewichtskonstanz eingedampft. Man erhält 46,9 g Rohprodukt in Form eines gelben Öls, welches nach Reinigung auf einer Chromatographiersäure (Kieselgel; Äther/n-Hexan) 37,1 g 3-(5-Hydroxy-3-methyl-1,3-pentadienyl)-2,4,4-trimethyl-2-cyclohexen-1-on ergibt.

10 g der so erhaltenen Substanz werden in 65 ml Methylenchlorid in einen 200-ml-Sulfierkolben, versehen mit Rührer, Thermometer, 25-ml-Tropftrichter mit Druckausgleich und einer Vorrichtung zur Inertbegasung eingebracht; hierauf wird das Reaktionsgefäss mit Argon gespült und während der ganzen Reaktionsdauer unter leichtem Argondruck gehalten. Danach wird die Lösung mit einem Eisbad auf 0 bis 5 °C gekühlt und anschliessend das Reaktionsgemisch bis zur Aufarbeitung in diesem Temperaturbereich gehalten.

So schnell es die Einhaltung dieser Reaktionstemperatur erlaubt, werden unter intensivem Rühren 13,0 ml Bromwasserstofflösung (63% in Wasser) zugetropft (in etwa 16 Minuten). Das Reaktionsgemisch wird dann noch während 20 Minuten nachgerührt.

Inzwischen werden ein 250-ml-Scheidetrichter ($S_1$) mit 110 ml 10-proz. Natriumchlorid und zwei weitere 250-ml-Scheidetrichter ($S_2$ + $S_3$) mit je 110 ml, insgesamt also mit 220 ml, 5-proz. Natriumbicarbonatlösung beschickt.

Zur Aufarbeitung werden nun das Reaktionsgemisch und anschliessend 45 ml Methylenchlorid

der Reihe nach und unter jeweiligem Schütteln durch die drei Extraktionsgefässe $S_1$–$S_3$ geschickt. Die beiden Methylenchloridphasen werden vereinigt, mit 0,4 ml 1,2-Butylenoxid versetzt, mit 20 g Natriumsulfat getrocknet, filtriert, mit 250 ml Äthylacetat nachgespült und das Filtrat im Rotationsverdampfer am Wasserstrahlvakuum bei einer Badtemperatur von 30 °C auf ein Volumen von etwa 50 ml eingeengt. Das Partialvakuum im Rotationsverdampfer wird mit Stickstoff entspannt, das Konzentrat mit 250 ml Äthylacetat versetzt und, wie beschrieben, erneut auf ein Volumen von 100 ml eingeengt, mit Stickstoff begast und dann die so erhaltene Lösung von 3-(5-Brom-3-methyl-1-cis, 3-trans-pentadienyl)-2,4,4-trimethyl-2-cyclohexen-1-on direkt für die Darstellung des Phosphoniumsalzes weiter verwendet.

In einem 350-ml-Sulfierkoben, versehen mit Rührer, Thermometer, 100-ml-Tropftrichter mit Druckausgleich und einer Vorrichtung zur Inertbegasung wird eine Lösung von 13,0 g Triphenylphosphin und 0,4 ml 1,2-Butylenoxid in 130 ml Äthylacetat vorgelegt. Das Reaktionsgefäss wird während der ganzen Reaktionsdauer mit Argon begast.

Bei Raumtemperatur wird nun unter Rühren die nach den obigen Angaben erhaltene Lösung des 3-(5-Brom-3-methyl-1-cis, 3-trans-pentadienyl)-2,4,4-trimethyl-2-cyclohexen-1-on im Verlaufe von 2 Stunden zugetropft.

Beim Auftreten der ersten Trübungserscheinungen im Reaktionsgemisch (nach etwa 10 Minuten) werden Impfkristalle zugesetzt. Das hellgelbe Phosphoniumsalz kristallisiert darauf laufend aus, und die Temperatur des Reaktionsgemisches steigt leicht (bis auf etwa 26 °C) an. Nach beendeter Zugabe des Bromides wird noch weitere 18 Stunden gerührt und dann das Produkt unter Stickstoff abgenutscht.

Das gelbliche Kristallisat wird unter Stickstoff auf der Nutsche 2mal mit je 60 ml Äthylacetat gewaschen und dann bei 50 °C im Trockenschrank am Wasserstrahlvakuum bis zur Gewichtskonstanz getrocknet. Man erhält 20,6 g [all-trans-3-Methyl-5-(2,6,6-trimethyl-3-oxo-1-cyclohexen-1-yl)-2,4-pentadienyl]-triphenylphosphoniumbromid.

In einem 350-ml-Sulfierkolben, versehen mit Rührer, 25-ml-Tropftrichter mit Druckausgleichrohr und einer Vorrichtung zur Inertbegasung, Thermometer und einem gefüllten Chlorcalciumrohr, werden unter Rühren und Argonbegasung in 105 ml Methylenchlorid, 20,6 g des nach den obigen Angaben erhaltenen Phosphoniumbromids und 2,8 g 2,7-Dimethyl-octatrien-(2,4,6)-dial-(1,8) gelöst. Die erhaltene Lösung wird unter fortgesetztem Rühren mit einem Eisbad auf 0–5 °C gekühlt und bei dieser Temperatur im Verlaufe von 11 Minuten tropfweise mit 10,0 ml Natriummethylatlösung (enthaltend 2,08 g $NaOCH_3$) versetzt.

Eine Stunde danach wird das Kühlbad entfernt und zum Reaktionsgemisch werden 0,4 ml Essigsäure getropft.

Nachher wird das Chlorcalciumrohr durch einen Destillationsaufsatz mit absteigendem Liebig-Kühler ersetzt. Unter Erwärmen mit einem Ölbad (62 °C) werden zuerst 19 ml Methylenchlorid abdestilliert. Danach werden bei fortgesetzter Destillation 127 ml Methanol in dem Masse durch den Tropftrichter zugetropft, dass das Volumen der Canthaxanthinlösung ungefähr konstant bleibt. Dabei kristallisiert das Canthaxanthin allmählich in metallisch-glänzenden Kristallen aus. Gleichzeitig steigt die Dampftemperatur von anfänglich 36 °C langsam bis auf 63 °C an (Ölbadtemperatur: 100 °C; Gesamtdestillat: 110 ml). Die so erhaltene Kristallsuspension wird noch heiss mit 12,7 ml Wasser versetzt und anschliessend während 17 Stunden bei Raumtemperatur gerührt.

Die Kristalle werden unter Argonbegasung abfiltriert und auf dem Filter 2mal mit je 40 ml 83-proz. Methanol gewaschen und gut abgepresst.

Das so erhaltene rohe Canthaxanthin kann erwünschtenfalls noch einer Isomerisierung (z.B. durch Erhitzen auf etwa 90 °C während etwa 15 Stunden) und einer Reinigung unterworfen werden, wobei man ein Produkt mit einem Schmelzpunkt von 199–203 °C (nicht korr.) erhält.

Beispiel 2

Das gemäss Beispiel 1 als Ausgangsmaterial verwendete 5-(2,6,6-Trimethyl-3-oxo-cyclohexen-1-yl)-3-methyl-2-trans-penten-4-in-1-ol kann wie folgt erhalten werden:

In einem 500 ml-Sulfierkolben, versehen mit Rührer, Kühlvorrichtung, Thermometer, Chlorcalciumrohr und einer Vorrichtung zum Arbeiten unter Inertgas, werden 16,8 g 2-Hydroxy-3,5,5-trimethyl-2-cyclohexen-1,4-dion, 100 ml Äthylenchlorid und 100 ml Ameisensäure eingebracht. Hierauf werden unter Rühren, portionsweise, innerhalb von 10 Minuten 26,2 g Zinkstaub zugegeben. Die Temperatur steigt hierbei von 15 °C auf 28 °C an. Anschliessend wird das Reaktionsgemisch mit einem Ölbad auf 75 °C erwärmt und bei dieser Temperatur während 5 ½ Stunden belassen. Zur Aufarbeitung wird das Reaktionsgemisch auf 15 °C abgekühlt und 10 Minuten lang gerührt. Hierauf wird das Reaktionsgemisch filtriert, der Zinkstaubrückstand am Filter 2mal mit je 150 ml Methylenchlorid gewaschen und die organische Phase in einem 1-Liter-Scheidetrichter $S_1$ gespült und mit 200 ml gesättigter Natriumchloridlösung beschickt. Zwei weitere 1-Liter-Scheidetrichter $S_2$ und $S_3$ werden mit je 200 ml gesättigter Natriumchloridlösung beschickt. Durch die drei Scheidetrichter $S_1$–$S_3$ werden der Reihe nach, jeweils unter guter Durchmischung, 2 Portionen zu je 300 ml Methylenchlorid geschickt. Die vereinigten organischen Phasen werden über 150 g Natriumsulfat getrocknet, filtriert und im Rotationsverdampfer am Wasserstrahlvakuum bei einer Badtemperatur von 50 °C bis zur Gewichtskonstanz eingedampft. Es werden 15,5 g hellgelbes, kristallines Rohprodukt erhalten, welches unter Erwärmen auf 65 °C in 45 ml Diisopropyläther gelöst wird. Nach dem Abkühlen auf etwa 40 °C wird während 18 Stunden bei − 20 °C stehengelassen. Die ausgefallenen weissen Kristalle werden abfiltriert, am Filter 2mal mit je 20 ml Diisopropyläther

(gekühlt auf −20 °C) gewaschen und dann im Vakuumtrockenschrank bei 40 °C bis zur Gewichtskonstanz getrocknet. Man erhält 14,9 g eines Produktes mit einem Schmelzpunkt von 113–115 °C, welches ein Tautomerengemisch von 3-Hydroxy-2,6,6-trimethyl-2-cyclohexen-1-on und 3-Hydroxy-2,4,4-trimethyl-2-cyclohexen-1-on darstellt.

15,4 g des so erhaltenen Tautomerengemisches in 150 ml Aceton werden zusammen mit 18,0 g Kaliumcarbonat in einen 350 ml Sulfierkolben eingebracht, welcher mit Rührer, Kühlvorrichtung, Chlorcalciumrohr und einer Vorrichtung zum Arbeiten unter Inertgas versehen ist. Hierauf werden bei 20 °C unter Rühren, innerhalb von etwa 5 Minuten 18,5 g Diäthylsulfat zugetropft. Anschliessend wird das Reaktionsgemisch auf einem Ölbad auf 56 °C erwärmt und während 18 Stunden bei dieser Temperatur belassen. Zur Aufarbeitung wird das Reaktionsgemisch auf 15 °C abgekühlt und hierauf im Rotationsverdampfer am Wasserstrahlvakuum bei einer Badtemperatur von 50 °C bis zur Gewichtskonstanz eingedampft. Der erhaltene gelbe Rückstand wird in 250 ml Wasser und 250 ml Methylenchlorid gelöst. Das so erhaltene Zweiphasengemisch wird in einen 1-Liter-Scheidetrichter $S_1$ gespült. Zwei weitere 1-Liter-Scheidetrichter $S_2$ und $S_3$ werden mit je 250 ml Wasser beschickt. Der Reihe nach und unter guter Durchmischung werden 2 Portionen zu je 250 ml Methylenchlorid durch die 3 Scheidetrichter $S_1$–$S_3$ geschickt. Die vereinigten organischen Phasen werden über 150 g Natriumsulfat getrocknet, filtriert und im Rotationsverdampfer am Wasserstrahlvakuum bei einer Badtemperatur von 50 °C bis zur Gewichtskonstanz eingedampft. Das so erhaltene Rohprodukt wird mittels Säulenchromatographie (Kieselgel, Laufmittel n-Hexan/Äther) gereinigt, wobei man 12,7 g 3-Äthoxy-2,6,6-trimethyl-2-cyclohexen-1-on erhält. In einen 500 ml Sulfierkolben, versehen mit Rührer, Thermometer, Kühlvorrichtung, Chlorcalciumrohr, Tropftrichter mit Druckausgleich und einer Vorrichtung zur Inertbegasung wird eine Lösung von 18,85 g Acetonmethyl-3-methyl-2-penten-4-inyl-acetal in 135 ml Tetrahydrofuran eingebracht. Unter starkem Rühren und Begasung mit Argon wird die Temperatur im Reaktionsgefäss mit Hilfe eines Kühlbades (−20 °C) auf −15 °C gesenkt und es werden hierauf im Verlaufe von 15 Minuten 66,6 ml einer 1,5M Butyllithiumlösung (6,4 g Butyllithium) derart zugetropft, dass die Temperatur des Reaktionsgemisches −7 °C nicht übersteigt. Anschliessend wird unter fortgesetztem Rühren und Begasung mit Argon im Verlaufe von 5 Minuten eine Lösung von 15,49 g des nach den obigen Angaben erhaltenen 3-Äthoxy-2,6,6-trimethyl-2-cyclohexen-1-on in 35 ml Tetrahydrofuran zugetropft. Die erhaltene hellgelbe, klare Lösung wird nach Entfernen des Kühlbades auf +10 °C aufgewärmt, insgesamt während $3^{11}/_{2}$ Stunden bei dieser Temperatur reagieren gelassen und dann tropfenweise, unter erneutem Kühlen (−20 °C) in dem Masse mit 75 ml 3N Schwefelsäure versetzt, dass die Temperaturen des Reaktionsgemisches 0 °C nicht übersteigt. Das so erhaltene zweiphasige Gemisch wird nach Entfernung des Kühlbades bei Raumtemperatur während 3 Stunden kräftig gerührt und dann in einen 1-Liter-Scheidetrichter $S_1$ gespült. Zwei weitere 1-Liter-Scheidetrichter $S_2$ und $S_3$ werden je mit 150 ml Äther beschickt und die wässrige Phase aus $S_1$ und anschliessend 3 Portionen zu je 100 ml gesättigter Natriumbicarbonatlösung der Reihe nach und unter jeweiligem Schütteln durch die 3 Scheidetrichter $S_1$–$S_3$ geschickt. Die wässrigen Phasen werden verworfen, die organischen Phasen vereinigt, mit 100 g Natriumsulfat getrocknet, filtriert und im Rotationsverdampfer am Wasserstrahlvakuum bei einer Badtemperatur von 40 °C bis zur Gewichtskonstanz eingeengt. Es werden 24,0 g eines hellgelben, kristallinen Rohproduktes erhalten, welches unter Erwärmen auf 65 °C in 50 ml Diisopropyläther gelöst wird. Nach dem Abkühlen auf 55 °C wird die erhaltene Lösung innerhalb einer Stunde auf Raumtemperatur abgekühlt und dann während weiteren 16 Stunden bei −20 °C stehengelassen. Die erhaltenen zitronenfarbigen, nadelförmigen Kristalle werden unter Argonbegasung abfiltriert, auf dem Filter 2mal mit je 20 ml Diisopropyläther (gekühlt auf −20 °C) gewaschen und dann im Vakuumtrockenschrank bei 40 °C bis zur Gewichtskonstanz getrocknet. Man erhält hierbei 5-(2,6,6-Trimethyl-3-oxo-cyclohexen-1-yl)-3-methyl-2-trans-penten-4-in-1-ol mit einem Schmelzpunkt von 103,5 °C–105,5 °C.

**Patentansprüche**

1. Verfahren zur Herstellung von Cyclohexenderivaten, dadurch gekennzeichnet, dass man eine Verbindung der Formel

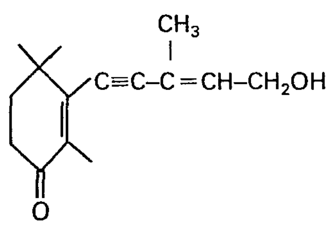

I

mittels Zink und Ameisensäure, Essigsäure oder Propionsäure hydriert und gewünschtenfalls die dabei erhaltene Verbindung der Formel

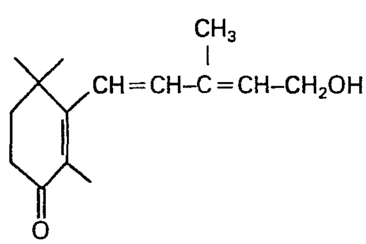

II

nach Überführung in ein Phosphoniumsalz der Formel

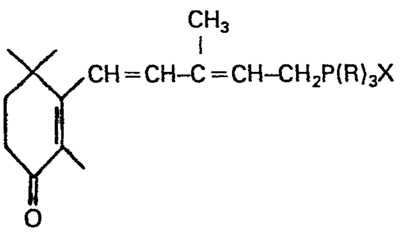

III

worin R Phenyl und X Chlor, Brom oder Jod bedeutet, in Canthaxanthin überführt.

2. Verfahren nach Anspruch 1, dadurch gekennzeichnet, dass man die Hydrierung mit Zink und Essigsäure durchführt.

**Revendications**

1. Procédé de préparation de dérivés de cyclohexène, caractérisé en ce qu'on hydrogène un composé de formule:

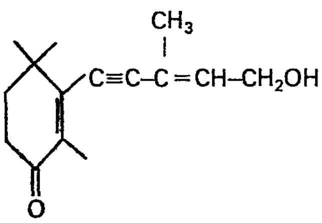

(I)

au moyen de zinc et d'acide formique, d'acide acétique ou d'acide propionique, et si on le désire en ce qu'on transforme le composé ainsi obtenu de formule:

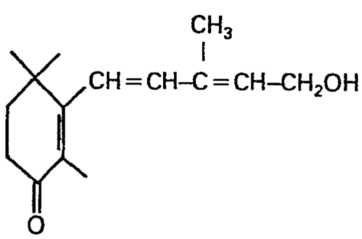

(II)

après transformation en un sel de phosphonium de formule:

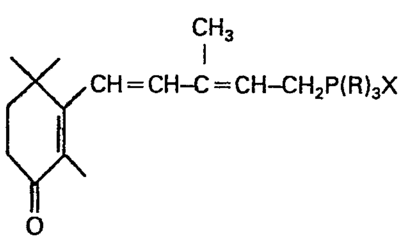

(III)

où R représente un phényle et X un chlore, un brome ou un iode en canthaxantine.

2. Procédé selon la revendication 1, caractérisé en ce qu'on conduit l'hydrogénation avec du zinc et de l'acide acétique.

**Claims**

1. Process for the manufacture of cyclohexene derivatives, characterized by hydrogenating a compound of the formula

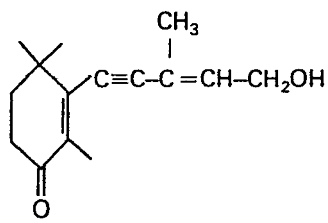

I

by means of zinc and formic acid, acetic acid or propionic acid and, if desired, converting the thereby obtained compound of the formula

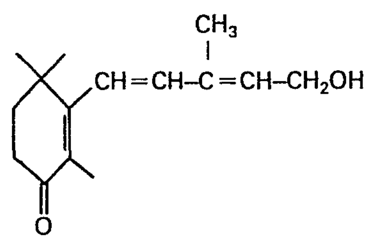

II

, after conversion into a phosphonium salt of the formula

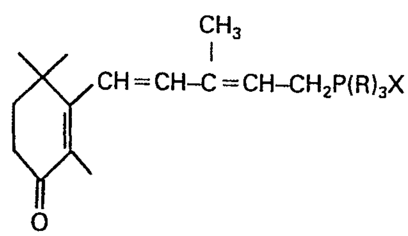

III

wherein R signifies phenyl and X signifies chlorine, bromine or iodine, into canthaxanthin.

2. Process according to claim 1, characterized in that the hydrogenation is carried out with zinc and acetic acid.